Europäisches Patentamt

European Patent Office

Office européen des brevets

(11) Veröffentlichungsnummer: **0 114 384**
**A2**

## (12) EUROPÄISCHE PATENTANMELDUNG

(21) Anmeldenummer: 83112998.6

(22) Anmeldetag: 22.12.83

(51) Int. Cl.³: **G 01 N 27/20**
G 01 N 33/44

(30) Priorität: 23.12.82 DE 3247852

(43) Veröffentlichungstag der Anmeldung:
01.08.84 Patentblatt 84/31

(84) Benannte Vertragsstaaten:
AT BE CH DE FR GB IT LI LU NL SE

(71) Anmelder: THYSSEN PLASTIK ANGER KG
Anzinger Strasse 1
D-8000 München 80(DE)

(72) Erfinder: Neueder, Dipl.-Ing.
Amselstrasse 9
D-8443 Furth(DE)

(54) Vorrichtung zum Prüfen von Kunststoffprofilen.

(57) Mit der Erfindung wird eine Prüfvorrichtung für Kunststoffprofile, vorzugsweise für extrudierte, strahlenvernetzte Polyäthylenrohre, vorgeschlagen, die jedoch anwendungsmäßig nicht auf dieses Gebiet beschränkt ist, wobei eine auf erhöhtem Spannungspotential stehende Elektrode und eine geerdete Elektrode vorgesehen sind, die das sich bewegende Kunststoffprofil außen umfassen und im Inneren des Profils, im Bereich der Vorrichtung, eine vorzugsweise magnetisch gehaltene, nach außen nicht direkt leitend geerdete Sonde aus vorzugsweise Metallgeflecht angeordnet ist. Die Feststellung von beispielsweise Elektronenlöchern in der Wandung des Kunststoffprofils wird erreicht, indem an diesen Orten Spannungsdurchschläge erfolgen. Mittels der erfindungsgemäß vorgeschlagenen Vorrichtung können selbstverständlich auch andere Fehlstellen, wie Lunker, Einschlüsse, Blasen usw. lokalisiert und durch Schaltbefehle entsprechender Geräte zur Markierung, Aussonderung usw. gegeben werden.

EP 0 114 384 A2

821202 PGm
TEP DS-ba
22. 12. 1982

## Vorrichtung zum Prüfen von Kunststoffprofilen

Die Erfindung bezieht sich auf eine Vorrichtung zum Prüfen von Kunststoffprofilen, vorzugsweise von vernetzten Rohren oder dergleichen.

Es ist bekannt, daß während der Extrusion von Kunststoffprofilen Lunker und Einschlüsse sowie bei nachgeschalteten Bearbeitungsverfahren, beispielsweise beim Vernetzen von Polyäthylen, in der Wand des Profiles Löcher auftreten können.

Besonders beim Strahlenvernetzen von Kunststoffprofilen treten Durchschüsse auf, die durch Elektronenbeschuß erzeugt werden und Profilwandungen von einigen Millimetern durchschlagen, wobei der Verlauf des Durchschußkanales nicht etwa geradlinig ist, sondern völlig unregelmäßigen Verlauf zeigen kann. Bisher wurde versucht, solche Löcher dadurch festzustellen, daß Profilabschnitte unter Wasser mit Luftdruck beaufschlagt werden und dann anhand der aufsteigenden Luftblasen die Durchschußkanäle visuell geortet werden können, worauf das beschädigte Profilstück ausgesondert wird. Andere Fehlstellen, wie beispielsweise Lunker oder Einschlüsse können mit diesem Prüfsystem jedoch nicht erfaßt werden. Auch diese vorgenannten Löcher, die in einem vernetzten Polyäthylenprofil immerhin etwa 1/10 mm breite Durchschußkanäle aufweisen, können durch diese Luftdruckprüfungen unter Wasser nur sehr bedingt lokalisiert werden. Legt man beispielsweise größere Rohrabschnitte, vor allem dünnere Rohrbündel in einen Wassertank ein und beaufschlagt diese mit Luftdruck, so kann es durchaus vorkommen, daß beispielsweise in unbeabsichtigt vorhandenen Biegestellen des Rohres vorhandene Durchschußkanäle durch diese Biegestelle abgedichtet sind und dadurch keine Luftblasen aufsteigen können. Man wendet deshalb bereits zwei verschiedene Methoden der Luftdruckprüfung an. Die erstere bezieht sich darauf, daß ein unter Druck stehender Profilstrang, beispielsweise Rohre, durch ein Wasserbad gezogen wird und das Abdrücken fertig gewickelter Rohrbündel mit beispielsweise Längen von 120, 200 und noch mehr Metern unter Wasser. Trotzdem kommt es vor, daß die beim Strahlenvernetzen entstandenen Durchschüsse, die ja nicht geradlinig, sondern im Zickzackkurs die Wand des Profiles durchdringen, nicht festgestellt werden können, weil entweder der Luftdruck oder der Biegeradius der gewickelten Rohrbündel die Eintritts- bzw. Austrittstrichter oder auch die Kanäle selbst im Werkstoff so zudrückt, daß keine Luftblasen austreten können (Es kann auch vorkommen, daß sich die Luftblasen in Hohlräumen innerhalb des Bündels sammeln und während der Prüfzeit nicht austreten). Daraus resultiert ein Anteil von nicht festgestellten Fehlstellen im Werkstoff, also ein Unsicherheitsfaktor, der nicht hingenommen werden kann.

Bei anderen Extrusionsprozessen wird zur Vernetzung des Werkstoffes beispielsweise ein chemisches Verfahren angewandt, bei dem die Zusätze z.B. Peroxyde u.a. entweder vor oder während des Extrusionsvorganges dem Ausgangsmaterial zugesetzt werden. Aus der Qualitätsprüfung solcherart hergestellter Kunststoffprofile ist es bekannt, mittels eines Hochspannungsprüfgerätes und einer im Rohr befindlichen Sonde die in leitender Verbindung mit dem Extrusionswerkzeug steht, also nach außen geerdet ist, Lunker oder andere Fehlstellen in der Profilwandung zu lokalisieren. Dabei schlägt an der Fehlstelle die Spannung vom Hochspannungsprüfgerät zu der nach außen direkt geerdeten Sonde im Inneren des Profiles durch.

Bei der Herstellung von Kabeln ist es bekannt, mit Hochspannungsprüfgeräten den Isolationswiderstand der Kunststoffisolierung zu prüfen. Dabei werden ebenfalls auftretende Lunker, Dünnstellen und andere Fehler in der Kabelisolierung festgestellt. Hierbei ist als Gegenpol der nach außen geerdete Draht im Inneren des Kabels herangezogen.

Für die Prüfung von strahlenvernetzten Kunststoffprofilen können die vorgenannten Prüfmethoden jedoch nicht angewandt werden. In der Regel werden nämlich die Kunststoffprofile, wobei es sich vorzugsweise um Rohre handelt, auf Trommeln in Längen von ungefähr 3.000, 4.000 und mehr Metern gewickelt. Anschließend in einem getrennten Arbeitsgang durch Bestrahlung vernetzt und dann während oder nach dem Rückwickeln auf die Fertiglängen von 120 - 200 m mittels Luftdruck geprüft. Dabei ist es technisch nicht möglich, von außen gehaltene und nach außen geerdete Metallsonden im Profilinneren zu fixieren.

Der Erfindung liegt daher die Aufgabe zugrunde, eine Vorrichtung zu besitzen, mit der die Prüfung von Kunststoffprofilen, insbesondere strahlenvernetzten Kunststoffrohren, auf Fehler in der Rohrwandung, besonders von Durchschußstellen, auf wirtschaftliche Weise möglich ist, das Prüfverfahren automatisierbar ist und mit der Nachteile der bisher bekannten Vorrichtungen und Verfahren zur Prüfung von Kunststoffprofilen mit Sicherheit vermieden werden.

Erfindungsgemäß wird dies dadurch erreicht, daß das sich bewegende Kunststoffrohr von einer ersten Elektrode und in entsprechendem Abstand davon von einer zweiten Elektrode umfaßt ist, wovon die eine Elektrode mit einer Spannungsquelle verbunden ist, die andere Elektrode geerdet und gegenüber der ersten Elektrode isoliert ist und eine Sonde im Inneren des Kunststoffrohres vorgesehen ist, die sich mindestens vom Anfang der ersten Elektrode bis zum Ende der zweiten Elektrode erstreckt und mittels eines Isolators an einem gehaltenen Schwimmer befestigt ist, von einem außen um den gleitenden Rohrstrang angeordneten Magneten.

Vorteilhafterweise bestehen die Elektroden aus feinen Metallfasern, die auf der Oberfläche des zu prüfenden Kunststoffprofiles gleiten und den Profilquerschnitt lückenlos umfangen.

Vorteilhafterweise bestehen die Elektroden aus engumfangenden Metallzylindern.

Vorteilhafterweise bestehen die Elektroden aus Lamellen oder anderen, den Profilumfang engumschließenden, elektrisch leitfähigen Materialien.

Vorteilhafterweise besteht die Sonde im Inneren des Rohrstranges aus einem elastischen, elektrisch leitfähigen Geflecht.

Vorteilhafterweise ist die Sonde im Inneren des Rohrstranges an der Oberfläche mit bürstenähnlichen Fasern versehen.

Vorteilhafterweise ist die Verbindung zwischen innenliegender Sonde und magnetisch gehaltenem Schwimmer ein Isolator.

Vorteilhafterweise ist der Magnethalter ein Dauermagnet.

Vorteilhafterweise ist der Magnethalter ein Elektromagnet.

Vorteilhafterweise ist eine Überwachungssonde für die im Rohrinneren gehaltene Sonde vorgesehen.

Die mit der Erfindung vorgeschlagene Prüfvorrichtung weist den Vorteil auf, daß auf wirtschaftliche Weise vollautomatisierbar, bisher nicht eindeutig auffindbare Fehlstellen der Wandung des zu prüfenden Profilstranges festgestellt werden können, wobei insbesondere Löcher und Durchschüsse mit Sicherheit lokalisiert werden.

Die mit der Erfindung vorgeschlagene Vorrichtung ist nachstehend anhand eines in der Abbildung dargestellten, schematisierten, Ausführungsbeispieles näher erläutert.

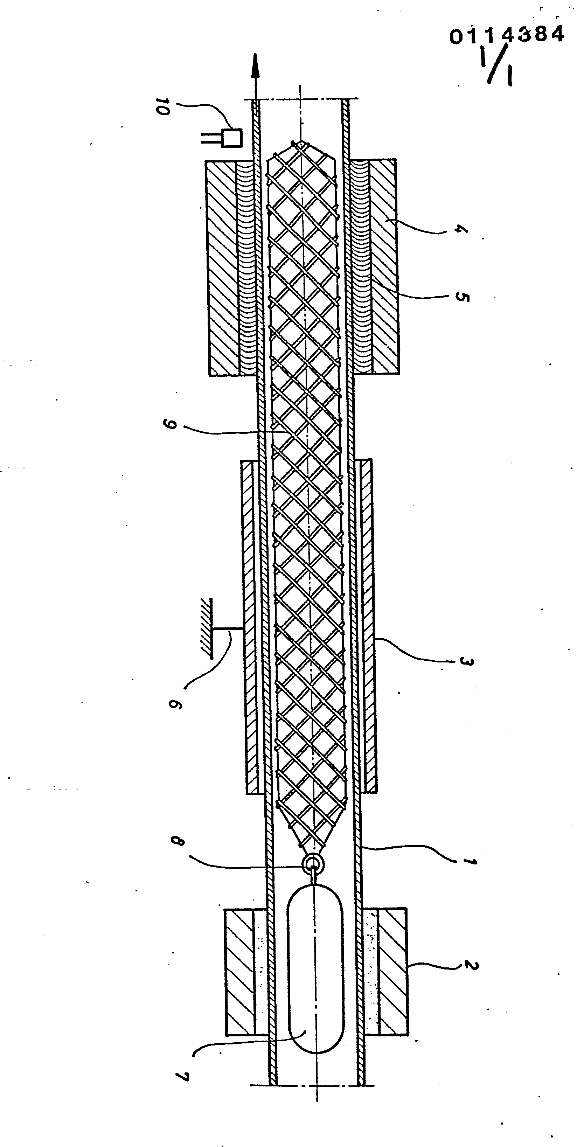

Die Figur 1 zeigt ein in Pfeilrichtung sich bewegendes Kunststoffprofil 1. Dieses Kunststoffprofil durchläuft in Pfeilrichtung zunächst einen Magnethalter 2, eine erste Elektrode 3 und nach kurzem Abstand eine weitere Elektrode, die mit der nicht dargestellten Spannungsquelle verbunden ist und außerdem mit Metallfasern 5 die Oberfläche am Anfang des Kunststoffprofilstranges 1 berührt. Mit 6 ist die Erdung der Elektrode 3 bezeichnet. Im Inneren des Profilstranges 1 ist ein Schwimmer 7 vorgesehen, der von dem Magneten 2, der den Rohrstrang außen umfaßt, gehalten wird und seinerseits über einen Isolator 8 eine Metallsonde 9 hält, die sich mindestens über die gesamte Länge der beiden Elektroden 3 und 4 nebst Zwischenraum erstreckt. Die Metallsonde 9 ist vorzugsweise aus einem elastischen, elektrisch leitfähigen Metallgeflecht gefertigt, so daß sie sich Unebenheiten und verschiedenen Durchmessern des Profilstranges 1 anpassen kann.

Mit 10 ist eine Überwachungssonde für die im Profilstrang gehaltene Metallsonde 9 bezeichnet, um anzuzeigen, falls sich der Schwimmer 7, aus welchen Gründen auch immer, von seinem Standort fortbewegt und damit die Metallsonde 9 aus dem Bereich der beiden Zylinder 3 und 4 entfernt.

Nicht dargestellt sind Anzeige- oder Folgevorrichtung, die im Störfall = Spannungsüberschlag in Betrieb gesetzt werden.

Die Erfindung geht davon aus, daß das für die Hochspannungsmessung im Inneren des Profilstranges 1 erforderliche Gegenpotential über einen "Kondensator" zu schaffen ist. Mit Rücksicht darauf, daß die Sonde 9 von außen nicht direkt gehalten werden kann, wird sie in bekannter Weise über einen Magnetschwimmer 7, der im Felde eines außen um das Profil herumgelegten Magneten 2 sich befindet, gehalten.

Um ein hinreichend hohes Potential aufbauen zu können, muß der Zylinder 3 eine entsprechend große Oberfläche bzw. entsprechende Länge besitzen. Der Luftspalt zwischen dem Inneren des Zylinders 4 und der äußeren Oberfläche des Kunststoffprofiles 1 soll möglichst gering sein. Als Hochspannungsquelle 4 können handelsübliche Hochspannungsgeneratoren eingesetzt werden.

Es hat sich herausgestellt, daß für die Anwendung der mit der Erfindung vorgeschlagenen Vorrichtung die nachstehenden Abmessungen, die als beispielsweise gelten, ein einwandfreies Funktionieren der Vorrichtung gewährleisten. Als Spannungsquelle dient ein Wechselspannungsgenerator von 25.000 Volt. Die Länge der Elektrode 4 beträgt etwa 30 cm, die Länge der Elektrode 3 ca. 50 cm. Der Luftspalt zwischen beiden beträgt etwa 20 cm. Die Länge der Sonde 9 etwa 100 cm. Profil: Rundprofil mit einem äußeren Durchmesser von 18 mm und einer Wanddicke von 2 mm. Werkstoff: Poläthylen, stahlvernetzt.

Durchläuft nun das Kunststoffprofil 1 in Pfeilrichtung die Vorrichtung und ist die Spannungsquelle eingeschaltet, so baut sich über die Sonde 9 ein elektrisches Feld zwischen den gegeneinander isolierten Elektroden 3 auf. Im Falle des Auftretens einer Fehlstelle im Bereich der Elektroden, beispielsweise eines Elektronenloches in der Wand des Kunststoffprofils 1, wird es zu einem Spannungsüberschlag von der Elektrode 3/4 zur Sonde 9 kommen. Dieser Spannungsüberschlag aktiviert ein Markierung- oder sonstiges Schaltgerät, wodurch die Fehlstelle registriert, markiert und später abgeschnitten oder anderweitig als funktionsuntüchtig gekennzeichnet wird. Durch weitere Schaltbefehle kann beispielsweise die Rückwickelanlage außer Betrieb gesetzt werden und dergleichen mehr.

Selbstverständlich ist die mit der Erfindung vorgeschlagene Vorrichtung nicht auf das in den Abbildungen dargestellte Ausführungsbeispiel beschränkt. Sie kann auch mit Vorteil auf anderen Gebieten, wo ähnliche Probleme auftauchen, eingesetzt werden.

821202 PGm
TEP DS-ba
22. 12. 1982

PATENTANSPRÜCHE
----------------

1.                                    Vorrichtung zum Prüfen
von Kunststoffprofilen, vorzugsweise von vernetzten Rohren
oder dergleichen, d a d u r c h   g e k e n n z e i c h -
n e t , d a ß  das sich bewegende Kunststoffrohr von einer
ersten geerdeten Elektrode (3) und in geringem Abstand davon
von einer zweiten Elektrode umfaßt ist, wovon die eine Elektrode mit einer Spannungsquelle verbunden ist, die andere
Elektrode geerdet und gegenüber der ersten Elektrode isoliert
ist und eine Sonde (9) im Inneren des Kunststoffrohres (1) vorgesehen ist, die sich mindestens vom Anfang der ersten Elektrode bis zum Ende der zweiten Elektrode erstreckt und mittels
eines Isolators an einem von einem außen um den gleitenden Rohrstrang angeordneten Magneten gehaltenen Schwimmer befestigt ist.

2.   Vorrichtung zum Prüfen von Kunststoffprofilen nach Anspruch 1,
     d a d u r c h   g e k e n n z e i c h n e t,  d a ß  die Sonde (9) im Inneren des Rohrstranges (1) aus einem elektrisch
     leitfähigen, elastischen Metallgeflecht besteht.

3.   Vorrichtung zum Prüfen von Kunststoffprofilen nach Anspruch
     1  ,   d a d u r c h   g e k e n n z e i c h n e t , d a ß

die Sonde (9) im Inneren des Rohres aus elektrisch leitfähigen Lamellen besteht.

4. Vorrichtung zum Prüfen von Kunststoffprofilen nach Anspruch 1 , d a d u r c h   g e k e n n z e i c h n e t ,   d a ß die Sonde (9) im Inneren des Rohres aus elektrisch leitfähigen Bürsten (5) besteht.

5. Vorrichtung zum Prüfen von Kunststoffprofilen nach Anspruch 1 - 4, d a d u r c h   g e k e n n z e i c h n e t , die Elektroden (3, 4) das Kunststoffprofil eng umfassende Metallrohre sind.

6. Vorrichtung zum Prüfen von Kunststoffprofilen nach Anspruch 1 - 4, d a d u r c h   g e k e n n z e i c h n e t ,   d a ß die Elektroden(3 u. 4) aus elektrisch leitfähigen, den äußeren Umfang des Rohres möglichst lückenlos umfassenden Bürsten, Lamellen oder anderen flexiblen Gebilden (5) bestehen.

7. Vorrichtung zum Prüfen von Kunststoffprofilen nach Anspruch 1 - 6, d a d u r c h   g e k e n n z e i c h n e t ,   d a ß der Magnethalter (2) ein Dauermagnet ist.

8. Vorrichtung zum Prüfen von Kunststoffprofilen nach Anspruch 1 - 6, d a d u r c h   g e k e n n z e i c h n e t ,   d a ß der Magnethalter (2) ein Elektromagnet ist.

9. Vorrichtung zum Prüfen von Kunststoffprofilen nach Anspruch 1 - 8, d a d u r c h   g e k e n n z e i c h n e t ,   d a ß die Verbindung von innenliegender Sonde (9) und magnetisch gehaltenem Schwimmer (7) ein Isolator (8) ist.

10. Vorrichtung zum Prüfen von Kunststoffprofilen nach Anspruch 1 - 9, dadurch gekennzeichnet, daß eine Überwachungssonde (10) für die im Rohrinneren gehaltene Sonde (9) vorgesehen ist.